# EUROPEAN PATENT SPECIFICATION

(11) **EP 2 540 693 B1**
(45) Date of publication and mention of the grant of the patent: **04.04.2018**
(21) Application number: 11747457.7
(22) Date of filing: 24.02.2011
(51) Int. Cl.: C07C 31/30, C07C 29/70, C08F 4/654, C08F 10/00, C07F 3/02

(54) **MIXED MAGNESIUM DIALKOXIDE PARTICULATE, METHOD FOR SYNTHESIZING SAME, AND METHOD FOR USE THEREOF**
MAGNESIUM-DIALKOXID-MISCHPARTIKEL, VERFAHREN ZU SEINER SYNTHETISIERUNG UND VERFAHREN ZU SEINER VERWENDUNG
DIALCOXYDE DE MAGNÉSIUM MIXTE PARTICULAIRE, PROCÉDÉ POUR SYNTHÉTISER CELUI-CI, ET PROCÉDÉ POUR L'UTILISATION DE CELUI-CI

(30) Priority: 25.02.2010 JP 2010039520
(43) Date of publication of application: 02.01.2013
(73) Proprietor: Colcoat Co., Ltd., Ota-ku Tokyo 143-0015 (JP)
(72) Inventor: YAMANAKA, Akihiko, Gyoda-shi Saitama 361-0033 (JP); KUMAI, Hiroshi, Gyoda-shi Saitama 361-0033 (JP)
(74) Representative: Ricker, Mathias
(86) International application number: PCT/JP2011/054160
(87) International publication number: WO 2011/105497

(56) References cited:
- EP-A1- 1 739 070
- EP-A1- 2 006 272
- WO-A1-2005/044873
- WO-A1-2005/102973
- WO-A1-2007/116815
- JP-A- 8 073 388
- JP-A- 2006 298 996
- JP-A- 2007 509 901
- JP-A- 2009 102 478
- JP-A- 2010 030 924
- JP-A- 2010 030 925

## Description

### Technical Field

The present invention relates to a method for synthesis of a magnesium dialkoxide particulate that is to be used for preparation of a solid catalyst component for olefin polymerization.

### Background Art

Magnesium diethoxide is used as solid catalyst component for polymerization of olefins such as propylene. Because the shape of polypropylene obtained by polymerization is analogous to the shape of the polymerization catalyst, it is common to use magnesium diethoxide which has a spherical or ellipsoid shape, and a mean particle size represented by D₅₀ of several tens of µm, and usually no greater than 60 µm, for balance with the strength of the polymerization catalyst.

Methods for synthesizing spherical or ellipsoid magnesium diethoxide by direct reaction between magnesium metal and ethyl alcohol are known in the prior art, as described in Patent Document 1 and elsewhere, but such methods cannot easily synthesize products with high bulk density, while large mean particle sizes have insufficient strength, and when used as solid catalyst components for polymerization of olefins, grinding and micronization during the preparation process can result in poor catalyst yield and increase the cost for the catalyst, such that despite the desire for excellent polymerization activity, it has become more common to employ inexpensive magnesium chloride as a solid catalyst component for polymerization of olefins. In addition, the strength of the catalyst itself tends to be insufficient, and problems tend to arise particularly when it is used in fluidized beds.

Patent Document 2 describes mixed magnesium dialkoxide, which is a mixture of magnesium metal diethoxide and another dialkoxide, but there is no mention regarding the bulk density of the mixed magnesium dialkoxide.

A similar mixed magnesium dialkoxide is described in Patent Document 3, and in the description of the examples, its bulk density is specified as being 0.30 or 0.31. Comparative Example 1 of the same patent mentions a bulk density of 0.41, but this is the bulk density of a ground magnesium diethoxide product. In addition, Patent Document 4 discloses spherical particles comprising magnesium alcoholates or a mixture of alcoholates and a process for their preparation.

### Prior Art Reference

### Patent Document

Patent Document 1: Japanese Examined Patent Publication HEI No. 7-20898
Patent Document 2: WO2005/102973
Patent Document 3: US Patent No. 6855656
Patent Document 4: WO2005/044873

### Disclosure of the Invention

### Problems to be Solved by the Invention

It is an object of the present invention to solve the problems outlined above by providing a novel method for synthesis of magnesium dialkoxide with high bulk density and with high breaking strength. It is another object of the invention to provide a catalyst component for polymerization of olefins that has higher polymerization activity than conventional magnesium diethoxide alone.

### Means for Solving the Problems

The invention provides a method for synthesis of a mixed magnesium dialkoxide particulate by direct solid-liquid reaction between particulate magnesium metal with a mean particle size of 50 µm to 500 µm and two alcohols, the two alcohols being ethyl alcohol and isopropyl alcohol and the molar ratio of the ethyl alcohol to isopropyl alcohol being 97-85:3-15 (with 100 as the total), wherein said mixed magnesium dialkoxide particulate is a mixture of magnesium diethoxide and magnesium diisopropoxide, and wherein the content of isopropoxide is 2.5 to 15 mol% of total ethoxides and isopropoxides, the mean particle size (D₅₀), measured using a Microtrac MT-3200 particle size analyzer, is 10 to 100 µm, and the bulk density, measured according to JIS K-51011-12-1(2004), is at least 0.4 g/ml.

The invention further provides a mixed magnesium dialkoxide particulate obtainable by the method of the present invention. Furthermore, the invention relates to the use of said mixed magnesium dialkoxide particulate as a catalyst component for polymerization of an olefin.

The alcohol mixture of two alcohols has a molar ratio between ethyl alcohol and the other alcohol, i.e. isopropyl alcohol, of 97-85:3-15 (total: 100), and especially 97-87:3-13 (total: 100). If the proportion of the other alcohol is not at least 3 mol%, the content of alkoxides other than diethoxide in the mixed magnesium dialkoxide will not reach 2.5 mol%, in which case the bulk density of the product will not be above 0.4 g/ml. Also, if the proportion of the other alcohol exceeds 15 mol%, aggregation of the product onto the particulates will be hampered, and it will not be possible to obtain a bulk density of 0.4 g/ml or greater.

The two alcohols are ethyl alcohol and isopropyl alcohol. The magnesium dialkoxide produced when using these two alcohols will be a mixture of magnesium diethoxide and magnesium diisopropoxide the product ratio being roughly proportional to the ratio of the alcohol mixture that is used.

The mean particle size of the magnesium metal is preferably 50 µm to 500 µm and especially 100 to 250 µm, and the particulate magnesium metal and the two alcohols comprising ethyl alcohol and isopropyl alcohol are used in a proportion for an ethyl alcohol content of 97-85 mol%, with a final addition ratio of magnesium metal/alcohols of = 1/3-30, as the molar ratio in the reaction system of the particulate magnesium metal and the alcohols, for direct reaction under reflux of the alcohol to produce a mixed magnesium dialkoxide particulate having an isopropoxide content of 2.5-13.0 mol% of the total alkoxides, particle shapes with a mean particle size (D₅₀) in the range of 20-80 µm, and a bulk density of 0.4 g/ml or greater.

The mixed magnesium dialkoxide particulate obtained in this manner may be used as a solid catalyst component for production of a polymerization catalyst for polyolefins, using a tetravalent titanium compound such as titanium tetrachloride and an electron donor such as dibutyl phthalate or cyclohexylmethyldimethoxysilane. An organic aluminum compound such as triethylaluminum is used therewith for polymerization of an olefin by slurry polymerization from an olefin gas.

### Effect of the Invention

Since the synthesis method of the invention uses a mixed alcohol to obtain high-strength magnesium dialkoxide, when it is used as an olefin polymerization catalyst it undergoes minimal breakdown during the preparation step and allows catalyst yield to be increased. Furthermore, the polymerization activity of the catalyst is increased by approximately 25% compared to conventional products, such that the polyolefin yield can be increased by 25%. Since the strength of the obtained polymerization catalyst is greater than with conventional products that employ magnesium diethoxide alone, it is possible to avoid disintegration during catalyst preparation by conventional methods, and as a result it can increase the polyolefin yield and prevent disintegration of the particle shapes of the obtained polyolefin. It is also possible to reduce catalyst loss and avoid trouble during fluidized bed polymerization. Modes for Carrying Out the Invention

The mean particle size of the magnesium metal used is most preferably 100 µm to 250 µm, and the particulate magnesium metal and the two alcohols comprising ethyl alcohol and isopropyl alcohol in a weight ratio for an ethyl alcohol molar ratio of 97-85%, are in a final addition ratio of magnesium metal/alcohols = 1/3-30 (molar ratio) in the reaction system of the particulate magnesium metal and the alcohols, for direct reaction under reflux of the alcohol to synthesize a mixed magnesium dialkoxide particulate having particle shapes with a mean particle size (D₅₀) in the range of 10-80 µm and preferably 20-80 µm, and a bulk density of 0.4 g/ml or greater.

According to the invention the bulk density of the mixed magnesium dialkoxide particulate is 0.4 or greater, preferably 0.41 or greater and even more preferably 0.42 or greater, and preferably no greater than 0.6 and even more preferably no greater than 0.5. A mixed magnesium dialkoxide particulate with a high bulk density has low grinding loss when it is used to produce a catalyst for olefin polymerization, and therefore provides an effect of increased catalyst product yield. Furthermore, due to the high strength of the polymerization catalyst, it is expected to be advantageous for vapor-phase polymerization of olefins.

The D₅₀ value referred to above is the particle size (µm) obtained in measurement of the particle size distribution of the particulate, when the integrated value of the particulate weight reaches 50 wt%, and the numerical value represents the median value of the particle sizes for the particulate. D₁₀ and D₉₀ similarly represent the particle size when the integrated value is 10% and 90%, respectively.

The magnesium metal used for the invention is most optimally a fine particulate material having a mean particle size (D₅₀) of 50-500 µm and a particle size distribution, represented by (D₉₀-D₁₀)/D₅₀, of no greater than 2. The metal is used in the form of a powder or shavings, but preferably, it is kept in an atmosphere of an inert gas such as nitrogen, with the metallic particle surfaces being protected as much as possible from surface oxidation or treated with a solvent that does not affect reaction of the metal surface, to minimize oxidation of the particle surfaces.

The alcohol used with ethyl alcohol is isopropyl alcohol.

Reaction between the magnesium metal and the two alcohols may be initiated by first adding a liquid mixture comprising the two alcohols in the prescribed ratio to a reaction system containing the magnesium metal, or by first adding only the ethyl alcohol to the reaction system, confirming start of the reaction by heat release or hydrogen release, and then adding the mixture of the two alcohols. The total amount of the two alcohols used is at a weight ratio of 3/1 to 30/1 with respect to the magnesium metal. If the amount of the two alcohols is less than 3 times the amount of magnesium the reaction will not proceed smoothly, unreacted magnesium metal may remain, and it will not be possible to control the particle size. If the amount exceeds 30 times, an excessive amount of alcohol will be present in the particles formed by the reaction, and removal of the alcohol during drying will create numerous voids inside the particles, resulting in particles with undesirably low bulk density.

The mixed magnesium dialkoxide particulate of the invention is composed of porous aggregates of spherical, ellipsoid, scaly or needle-like mixed magnesium dialkoxide primary particless with a particle size of 1-10 µm.

A catalyst is preferably used in the synthesis reaction for the magnesium dialkoxide of the invention. The catalyst used may be an alkyl halide, a metal halide, or iodine. It is suitably used at 0.1-20.0 mass% and preferably 0.5-10.0 mass% with respect to the magnesium metal. The catalyst is optimally added at the start of the reaction, but the reaction may also be conducted with addition of the catalyst in combination with divided portions of the starting materials.

The magnesium metal and the two alcohols may be added to the reaction system either in divided portions or in a continuous manner. Addition may be made over a period from 10 minutes to 1200 minutes with an appropriate number of portions added, but the addition is preferably divided over 5 portions, and addition of the alcohol other than ethyl alcohol is preferably completed by the half-point of the total reaction time. The divided portions are added to the reaction under reflux of the alcohol. The proportion of each addition may be as desired.

The reaction is conducted under reflux of the alcohol. The stirring speed is between 20 rpm and 600 rpm, although this will differ depending on the desired particle size and bulk density. The reaction time is from 10 minutes to 1200 minutes, including the time for addition of the starting materials. The preferred reaction time is between 60 minutes to 240 minutes, including the time for addition of the starting materials. The reaction may be judged to be complete when no further hydrogen is generated, but preferably the reaction mixture is stirred for maturation for 30-90 mins in a temperature range of 50°C to 120°C after completion of the reaction.

### Examples

The present invention will now be explained in greater detail by the following examples, with the understanding that these examples are in no way limitative on the invention. The "parts" refer to parts by weight. "IPA" stands for isopropyl alcohol, and "n-PA" stands for n-propyl alcohol.

### Example 1

After thorough N₂ substitution of a reactor equipped with an H₂ flow rate gas meter, reflux condenser, thermometer and stirrer, 200.0 parts of ethyl alcohol and 27.0 parts of IPA were added and the mixture was stirred at 100-300 rpm, room temperature. After stabilization of the rotational speed, there were added, as a catalyst, 3.0 parts of iodine and 6.0 parts of magnesium metal starting material with 50.0 parts of ethyl alcohol while stirring, which was continued for an additional 30 minutes at room temperature. An oil bath was used for heating to raise the temperature, and reaction was conducted for 15 minutes under reflux of the alcohol. Next, 4.8 parts of magnesium metal and 20.0 parts of ethyl alcohol were added in 5 portions at 20 to 3 minute intervals, with constant stirring and temperature conditions for reaction for 100 minutes under alcohol reflux, and upon confirming cessation of H₂ production, 50.0 parts of ethyl alcohol was added to complete the reaction. The total amount of magnesium metal added was 30.0 parts, the total amount of ethyl alcohol added was 400 parts (95.0 mol%), the total amount of IPA added was 27.0 parts (5.0 mol%), and the total reaction time was 180 minutes. The obtained reaction mixture was transferred to a rotary evaporator, and the ethyl alcohol was distilled off under conditions of 60°C, 100 mmHg to obtain 121.3 parts of dried mixed magnesium dialkoxide. The obtained particulate consisted of particles with D₅₀ of 53.6 µm, D₁₀ of 18.4 µm and D₉₀ of 251.4 µm, and the particle size distribution, represented by (D₉₀-D₁₀)/D₅₀, was 4.35. The bulk density was 0.456 g/ml (measured according to JIS K-51011-12-1(2004)). The particle sizes and the particle size distribution were measured using a MICROTRAC MT-3200 (product of Nikkiso Co., Ltd.).

### Example 2

After sufficient N₂ substitution of a reactor equipped with an H₂ flow rate gas meter, reflux condenser, thermometer and stirrer, 180.0 parts of ethyl alcohol was added and the mixture was stirred at 100-300 rpm, room temperature. After stabilization of the rotational speed, there were added, as a catalyst, 3.0 parts of iodine and 6.0 parts of magnesium metal starting material with 50.0 parts of ethyl alcohol while stirring, which was continued for an additional 30 minutes at room temperature. An oil bath was used for heating to raise the temperature, and reaction was conducted for 15 minutes under reflux of the alcohol. Next, with constant stirring and temperature conditions, 4.8 parts of magnesium metal, 0.4 part of ethyl alcohol and 27.0 parts of IPA were added, and then 4.8 parts of magnesium metal and 20.0 parts of ethyl alcohol were added, in 4 portions at 20 to 3 minute intervals for reaction for 100 minutes under alcohol reflux, and upon confirming cessation of H₂ production, 50.0 parts of ethyl alcohol was added to complete the reaction. The total amount of magnesium metal added was 30.0 parts, the total amount of ethyl alcohol added was 400 parts (95.0 mol%), the total amount of IPA added was 27.0 parts (5.0 mol%), and the total reaction time was 180 minutes. The obtained reaction mixture was transferred to a rotary evaporator, and the ethyl alcohol was distilled off under conditions of 60°C, 100 mmHg to obtain 122.1 parts of dried mixed magnesium dialkoxide. The obtained particulate consisted of particles with D₅₀ of 40.5 µm, D₁₀ of 20.3 µm and D₉₀ of 167.5 µm, and the particle size distribution, represented by (D₉₀-D₁₀)/D₅₀, was 3.63. The bulk density was 0.437 g/ml (measured according to JIS K-51011-12-1(2004)). The particle sizes and particle size distribution were measured using a MICROTRAC MT-3200 (product of Nikkiso Co., Ltd.).

### Example 3 (not according to the invention)

After thorough N₂ substitution of a reactor equipped with an H₂ flow rate gas meter, reflux condenser, thermometer and stirrer, 200.0 parts of ethyl alcohol and 27.0 parts of n-PA were added and the mixture was stirred at 100-300 rpm, room temperature. After stabilization of the rotational speed, there were added, as a catalyst, 3.0 parts of iodine and 6.0 parts of magnesium metal starting material with 50.0 parts of ethyl alcohol while stirring, which was continued for an additional 30 minutes at room temperature. An oil bath was used for heating to raise the temperature, and reaction was conducted for 15 minutes under reflux of the alcohol. Next, 4.8 parts of magnesium metal and 20.0 parts of ethyl alcohol were added in 5 portions at 20 to 3 minute intervals, with constant stirring and temperature conditions for reaction for 100 minutes under alcohol reflux, and upon confirming cessation of H₂ production, 50.0 parts of ethyl alcohol was added to complete the reaction. The total amount of magnesium metal added was 30.0 parts, the total amount of ethyl alcohol added was 400.0 parts (5.0 mol%), the total amount of n-PA added was 27.0 parts (5.0 mol%), and the total reaction time was 180 minutes. The obtained reaction mixture was transferred to a rotary evaporator, and the ethyl alcohol was distilled off under conditions of 60°C, 100 mmHg to obtain 120.1 parts of dried mixed magnesium dialkoxide. The obtained particulate consisted of particles with D₅₀ of 45.6 µm, D₁₀ of 20.4 µm and D₉₀ of 200.4 µm, and the particle size distribution, represented by (D₉₀-D₁₀)/D₅₀, was 3.94. The bulk density was 0.466 g/ml (measured according to JIS K-51011-12-1(2004)). The particle sizes and the particle size distribution were measured using a MICROTRAC MT-3200 (product of Nikkiso Co., Ltd.).

### Example 4

After thorough N₂ substitution of a reactor equipped with an H₂ flow rate gas meter, reflux condenser, thermometer and stirrer, 205.9 parts of ethyl alcohol and 19.2 parts of IPA were added and reaction was conducted in the same manner as Example 1. The total amount of ethyl alcohol added was 405.9 parts (96.5 mol%), and the total amount of IPA added was 19.2 parts (3.5 mol%). The obtained particulate consisted of particles with D₅₀ of 21.6 µm, D₁₀ of 6.7 µm and D₉₀ of 90.8 µm, and the particle size distribution, represented by (D₉₀-D₁₀)/D₅₀, was 3.90. The bulk density was 0.461 g/ml (measured according to JIS K-51011-12-1(2004)). The particle sizes and the particle size distribution were measured using a MICROTRAC MT-3200 (product of Nikkiso Co., Ltd.).

### Example 5

After thorough N₂ substitution of a reactor equipped with an H₂ flow rate gas meter, reflux condenser, thermometer and stirrer, 178.6 parts of ethyl alcohol and 55.0 parts of IPA were added and reaction was conducted in the same manner as Example 1. The total amount of ethyl alcohol added was 378.6 parts (90.0 mol%), and the total amount of IPA added was 55.0 parts (10.0 mol%). The obtained particulate consisted of particles with D₅₀ of 33.7 µm, D₁₀ of 10.1 µm and D₉₀ of 237.0 µm, and the particle size distribution, represented by (D₉₀-D₁₀)/D₅₀, was 6.70. The bulk density was 0.419 g/ml (measured according to JIS K-51011-12-1(2004)). The particle sizes and the particle size distribution were measured using a MICROTRAC MT-3200 (product of Nikkiso Co., Ltd.).

### Comparative Example 1

After sufficient N₂ substitution of a reactor equipped with an H₂ flow rate gas meter, reflux condenser, thermometer and stirrer, 149.7 parts of ethyl alcohol was added and the mixture was stirred at 100-300 rpm, room temperature. After stabilization of the rotational speed, there were added, as a catalyst, 2.3 parts of iodine and 5.0 parts of magnesium metal starting material with 34.0 parts of ethyl alcohol while stirring, which was continued for an additional 30 minutes at room temperature. An oil bath was used for heating to raise the temperature, and reaction was conducted for 15 minutes under reflux of the alcohol. Next, 4.0 parts of magnesium metal and 17.0 parts of ethyl alcohol were added in 5 portions at 20 to 3 minute intervals, with constant stirring and temperature conditions for reaction for 100 minutes under alcohol reflux, and upon confirming cessation of H₂ production, 32.7 parts of ethyl alcohol was added to complete the reaction. The total amount of magnesium metal added was 25.0 parts, the total amount of ethyl alcohol added was 334.1 parts, and the total reaction time was 180 minutes. The obtained reaction mixture was transferred to a rotary evaporator, and the ethyl alcohol was distilled off under conditions of 60°C, 100 mmHg to obtain 120.1 parts of dried mixed magnesium diethoxide. The obtained magnesium diethoxide consisted of particles with D₅₀ of 42.1 µm, D₁₀ of 27.9 µm and D₉₀ of 95.4 µm, and the particle size distribution, represented by (D₉₀-D₁₀)/D₅₀, was 1.60. The bulk density was 0.319 g/ml (measured according to JIS K-51011-12-1(2004)). The particle sizes and the particle size distribution were measured using a MICROTRAC MT-3200 (product of Nikkiso Co., Ltd.).

### Comparative Example 2

After thorough N₂ substitution of a reactor equipped with an H₂ flow rate gas meter, reflux condenser, thermometer and stirrer, 82.8 parts of ethyl alcohol and 87.1 parts of IPA were added and the mixture was stirred at 100-300 rpm, room temperature. After stabilization of the rotational speed, there were added, as a catalyst, 2.3 parts of iodine and 5.0 parts of magnesium metal starting material while stirring, which was continued for an additional 30 minutes at room temperature. An oil bath was used for heating to raise the temperature, and reaction was conducted for 15 minutes while circulating the alcohol. Next, 4.0 parts of magnesium metal and 17.0 parts of ethyl alcohol were added over 5 portions at 20 to 3 minute intervals, with constant stirring and temperature conditions for reaction for 100 minutes under alcohol reflux, and upon confirming cessation of H₂ production, 65.4 parts of ethyl alcohol was added to complete the reaction. The total amount of magnesium metal added was 25.0 parts, the total amount of ethyl alcohol added was 267.2 parts (80.0 mol%), the total amount of IPA added was 87.1 parts (20.0 mol%), and the total reaction time was 180 minutes. The obtained reaction mixture was transferred to a rotary evaporator, and the ethyl alcohol was distilled off under conditions of 60°C, 100 mmHg to obtain 129.1 parts of dried mixed magnesium dialkoxide. The obtained magnesium dialkoxide was almost totally aggregated, making it impossible to measure the particle size distribution and bulk density.

### Comparative Example 3

After thorough N₂ substitution of a reactor equipped with an H₂ flow rate gas meter, reflux condenser, thermometer and stirrer, 212.3 parts of ethyl alcohol and 11.0 parts of IPA were added and reaction was conducted in the same manner as Example 1. The total amount of ethyl alcohol added was 412.3 parts (98.0 mol%), and the total amount of IPA added was 11.0 parts (2.0 mol%). The obtained particulate were particles with D₅₀ of 32.7 µm, D₁₀ of 11.9 µm and D₉₀ of 158.4 µm, and the particle size distribution, represented by (D₉₀-D₁₀)/D₅₀, was 4.50. The bulk density was 0.287 g/ml (measured according to JIS K-51011-12-1(2004)). The particle sizes and the particle size distribution were measured using a MICROTRAC MT-3200 (product of Nikkiso Co., Ltd.).

### Working Example

### (Preparation of catalyst component)

1) After adding 10.0 parts of each of the magnesium dialkoxides obtained in Examples 1 to 5 and Comparative Examples 1 and 3, and 80 ml of toluene in a reactor equipped with a cooler and stirrer that had been sufficiently substituted with N₂, 20 ml of titanium tetrachloride was added while stirring, and stirring was continued with heating to 90°C.
2) Next, 2 .7 ml of dibutyl phthalate was added at 90°C and reaction was conducted for 2 hours. The stirring was suspended to remove the supernatant liquid.
3) A procedure of adding 100 ml of toluene and stirring for 10 minutes and then suspending the stirring to remove the supernatant was repeated twice.
4) Next, 80 ml of toluene and 20 ml of titanium tetrachloride were added to the reactor, and reaction was conducted at 90°C for 2 hours. After the reaction, stirring was suspended, the mixture was allowed to stand for 10 minutes, the supernatant liquid was removed, and the temperature was returned to 40°C.
5) A procedure of adding 200 ml of heptane and stirring for 10 minutes, allowing the mixture to stand and removing the supernatant, was repeated 10 times. A 250 ml portion of heptane was added to obtain a solid catalyst component.

### (Propylene polymerization)

The catalytic activity was calculated from the Ti loading ratio of the catalyst and the amount of polymer obtained.
1) After adding 185 ml of heptane to a thoroughly N₂-substituted reactor equipped with a cooler and stirrer, the mixture was heated to 60°C in an oil bath. When a temperature of 60°C was reached, 10 ml of triethylaluminum and 1 ml of cyclohexylmethyldimethoxysilane were added, and 5.0 ml of a solid catalyst component prepared using one of the magnesium dialkoxides of Examples 1 to 5 and Comparative Examples 1 and 3 was further added.
2) Polymerization was conducted for 15 minutes with a propylene gas flow rate of 2.0 L/min. The propylene gas was cut off after 15 minutes, and ethanol/hydrochloric acid (1/3) was added to suspend the polymerization.
3) Approximately 200 ml of purified water was added 3 times for washing treatment, and the obtained polypropylene was dried. The dried polypropylene yield was measured. The results are shown in Table 1.

**Table 1**

| Examples and Comparative Examples | Mixed alcohol | | Polymerization results |
|---|---|---|---|
| | Name | Mixing ratio (mol%) | Catalyst activity (g/mmol-Ti ·g) |
| Example 1 | Ethanol, IPA | Ethanol 95.0% | 475.4 |
| | | IPA 5.0% | |
| 2 | Ethanol, IPA | Ethanol 95.0% | 496.5 |
| | | IPA 5.0% | |
| (non-inventive) 3 | Ethanol, n-PA | Ethanol 95.0% | 502.5 |
| | | n-PA 5.0% | |
| 4 | Ethanol, IPA | Ethanol 96.5% | 501.4 |
| | | IPA 3.5% | |
| 5 | Ethanol, IPA | Ethanol 90.0% | 491.5 |
| | | IPA 10.0% | |
| Comp. Ex. 1 | Ethanol alone | Ethanol 100.0% | 395.2 |
| Comp. Ex. 2 | Ethanol, IPA | Ethanol 80.0% | - |
| | | IPA 20.0% | |
| Comp. Ex. 3 | Ethanol, IPA | Ethanol 98.0% | 382.5 |
| | | IPA 2.0% | |

Table 1 shows that the mixed magnesium dialkoxides exhibited catalytic activity with values of about 100 gram/mmol-Ti-h, which were higher than with magnesium diethoxide alone. It was not possible to prepare a polymerization catalyst in Comparative Example 2.

The propoxide groups in each mixed magnesium dialkoxide were measured, and comparison was made between the amount of IPA or n-PA actually added and the number of corresponding groups in the mixed magnesium dialkoxide that was produced. To 3.0 g of the mixed magnesium dialkoxide and magnesium diethoxide there was added 57.4 g of ion-exchanged water, and the mixture was stirred. After thorough stirring, 5.2 g of concentrated nitric acid was added and stirring was continued for 1 hour for hydrolysis. The reaction mixture was filtered and analyzed with a GC-2014 by Shimadzu Corp.

**Table 2**

| Examples and Comparative Examples | IPA content or n-PA content (mol%) |
|---|---|
| Example 1 | 4.5 |
| Example 2 | 4.3 |
| Example 3 (non-inventive) | 4.1 |
| Example 4 | 3.0 |
| Example 5 | 8.9 |
| Comp. Example 1 | 0 |
| Comp. Example 2 | - |
| Comp. Example 3 | 1.2 |

Table 2 shows that when the amount of IPA or n-PA used is within the range specified in the present claims, about 80-90% of the added starting materials react to produce propoxide groups in the mixed magnesium dialkoxide, but with addition below this range, the reactivity with magnesium decreases and the number of alkoxide groups other than ethoxide introduced into the dialkoxide is also reduced.

### Industrial Applicability

The invention provides a magnesium dialkoxide particulate that is to be used for preparation of a solid catalyst component for olefin polymerization, and is therefore industrially useful.

## Claims

1. A method for synthesis of a mixed magnesium dialkoxide particulate by direct solid-liquid reaction between particulate magnesium metal with a mean particle size of 50 µm to 500 µm and two alcohols, the two alcohols being ethyl alcohol and isopropyl alcohol and the molar ratio of the ethyl alcohol to isopropyl alcohol being 97-85:3-15 (with 100 as the total), wherein said mixed magnesium dialkoxide particulate is a mixture of magnesium diethoxide and magnesium diisopropoxide, and wherein the content of isopropoxide is 2.5 to 15 mol% of total ethoxides and isopropoxides, the mean particle size (D₅₀), defined and measured as disclosed in present specification, is 10 to 100 µm, and the bulk density, measured according to JIS K-51011-12-1(2004), is at least 0.4 g/ml.

2. The method of claim 1, wherein the particulate magnesium metal has a mean particle size of 100 µm to 250 µm, the two alcohols are used in a proportion for an ethyl alcohol content of 97-85 mol%, with a final addition ratio of magnesium metal to alcohols of 1:3 to 1:30, as the molar ratio in the reaction system comprising the particulate magnesium metal and the two alcohols, and the reaction is conducted under reflux of the alcohol, to produce a mixed magnesium dialkoxide particulate having an isopropoxide content of 2.5 to 13.0 mol% of total ethoxides and isopropoxides, particle shapes with a mean particle size (D₅₀), defined and measured as disclosed in present specification, in the range of 20 to 80 µm, and a bulk density, measured according to JIS K-51011-12-1(2004), of at least 0.4 g/ml.

3. A mixed magnesium dialkoxide particulate obtainable by the method according to claim 1 or 2.

4. Use of a mixed magnesium dialkoxide particulate according to claim 3 as a catalyst component for polymerization of an olefin.

## Patentansprüche

1. Verfahren zur Synthese eines Magnesiumdialkoxid-Mischpartikels mittels direkter Fest-Flüssig-Reaktion zwischen teilchenförmigem Magnesiummetall mit einer durchschnittlichen Teilchengröße von 50 µm bis 500 µm und zwei Alkoholen, wobei die zwei Alkohole Ethanol und Isopropanol sind und das Molverhältnis von Ethanol zu Isopropanol 97-85:3-15 (mit 100 als Gesamtsumme) beträgt, wobei das Magnesiumdialkoxid-Mischpartikel ein Gemisch aus Magnesiumdiethoxid und Magnesiumdiisopropoxid ist, und wobei der Gehalt an Isopropoxid 2,5 bis 15 Mol-% der gesamten Ethoxide und Isopropoxide beträgt, wobei die durchschnittliche Teilchengröße (D₅₀), definiert und bestimmt gemäß der vorliegenden Beschreibung, 10 bis 100 µm beträgt und die Schüttdichte, bestimmt gemäß JIS K-51011-12-1(2004), mindestens 0,4 g/ml beträgt.

2. Verfahren nach Anspruch 1, wobei das teilchenförmige Magnesiummetall eine durchschnittliche Teilchengröße von 100 µm bis 250 µm aufweist, die zwei Alkohole in einem Verhältnis für einen Ethanolgehalt von 97-85 Mol-%, mit einem finalen Zugabeverhältnis von Magnesiummetall zu Alkoholen 1:3 bis 1:30, als Molverhältnis in dem Reaktionssystem umfassend das teilchenförmige Magnesiummetall und die zwei Alkohole, verwendet werden, und die Reaktion unter Rückfluss des Alkohols durchgeführt wird, um ein Magnesiumdialkoxid-Mischpartikel mit einem Isopropoxidgehalt von 2,5 bis 13,0 Mol-% der gesamten Ethoxide und Isopropoxide, Teilchenformen mit einer durchschnittlichen Teilchengröße (D₅₀), definiert und bestimmt gemäß der vorliegenden Beschreibung, im Bereich von 20 bis 80 µm, und einer Schüttdichte, bestimmt gemäß JIS K-51011-12-1(2004), von mindestens 0,4 g/ml zu ergeben.

3. Magnesiumdialkoxid-Mischpartikel, erhältlich durch das Verfahren gemäß Anspruch 1 oder 2.

4. Verwendung eines Magnesiumdialkoxid-Mischpartikels gemäß Anspruch 3 als Katalysatorkomponente zum Polymerisieren eines Olefins.

## Revendications

1. Procédé de synthèse d'une particule de dialcoxydes de magnésium mixtes par réaction solide-liquide directe entre un métal de magnésium particulaire ayant une granulométrie moyenne de 50 µm à 500 µm et deux alcools, les deux alcools étant l'alcool éthylique et l'alcool isopropylique et le rapport molaire de l'alcool éthylique à l'alcool isopropylique étant de 97 à 85:3 à 15 (100 au total), dans lequel ladite particule de dialcoxydes de magnésium mixtes est un mélange de diéthoxyde de magnésium et de diisopropoxyde de magnésium, et dans lequel la teneur en isopropoxyde est de 2,5 à 15 % en moles des éthoxydes et isopropoxydes totaux, la granulométrie moyenne (D₅₀), définie et mesurée comme décrit dans la présente demande, est de 10 à 100 µm, et la densité apparente, mesurée selon la norme JIS K-51011-12-1(2004), est d'au moins 0,4 g/ml.

2. Procédé selon la revendication 1, dans lequel le métal de magnésium particulaire a une granulométrie moyenne de 100 µm à 250 µm, les deux alcools sont utilisés en une proportion pour une teneur en alcool éthylique de 97 à 85 % en moles, avec un rapport d'addition final du métal de magnésium aux alcools de 1:3 à 1:30, en tant que rapport molaire du système réactionnel comprenant le métal de magnésium particulaire et les deux alcools, et la réaction est réalisée au reflux de l'alcool, pour produire une particule de dialcoxydes de magnésium mixtes ayant une teneur en isopropoxyde est de 2,5 à 13,0 % en moles des éthoxydes et isopropoxydes totaux, une forme des particules ayant une granulométrie moyenne (D₅₀), définie et mesurée comme décrit dans la présente demande, comprise dans la plage allant de 20 à 80 µm, et une densité apparente, mesurée selon la norme JIS K-51011-12-1(2004), d'au moins 0,4 g/ml.

3. Particule de dialcoxydes de magnésium mixtes pouvant être obtenue par le procédé selon la revendication 1 ou 2.

4. Utilisation d'une particule de dialcoxydes de magnésium mixtes selon la revendication 3 en tant que composant catalytique pour la polymérisation d'une oléfine.
